# EUROPEAN PATENT APPLICATION

(11) **EP 4 483 942 A1**
(43) Date of publication of application: **01.01.2025**
(21) Application number: 22929042.4
(22) Date of filing: 12.07.2022
(51) Int. Cl.: A61N 1/36, A61N 1/04

(54) **ELECTRICAL MUSCLE STIMULATION SYSTEM AND ELECTRICAL MUSCLE STIMULATION METHOD CAPABLE OF CONTROLLING VARIABLE CHANNEL**

(30) Priority: 26.02.2022 KR 20220025598
(71) Applicant: Coremovement Co., Ltd., Busan 48548 (KR)
(72) Inventor: KIM, Myeongcheol, Busan 48299 (KR)
(74) Representative: Schmitt-Nilson Schraud Waibel Wohlfrom Patentanwälte Partnerschaft mbB
(86) International application number: PCT/KR2022/010094
(87) International publication number: WO 2023/163291

(57) **Abstract**

Disclosed are an electrical muscle stimulation system and an electrical muscle stimulation method capable of controlling a variable channel, by which control signals are transmitted to a plurality of electrode channels through individual channels, so that electrical muscle stimulation is applied to a specific human body part. The electrical muscle stimulation system includes a plurality of electrode pads which are disposed on a main body disposed to be wearable by a user or disposed to be able to be in contact with the body of the user, and spaced apart from each other at locations corresponding to various human body parts; and a control unit which outputs a plurality of control signals to the plurality of electrode pads through a plurality of individual channels and individually controls the plurality of electrode pads according to the plurality of control signals. The control unit determines a control signal by selecting one or more working electrode pads from among the plurality of electrode pads according to a target location and a target intensity of electrical muscle stimulation to be caused to the body of the user.

## Description

### [TECHNICAL FIELD]

Embodiments of the present disclosure described herein relate to an electrical muscle stimulation system, and more particularly, relate to an electrical muscle stimulation system capable of controlling a variable channel and an electrical muscle stimulation method.

### [BACKGROUND ART]

Electrical muscle stimulation (EMS) devices have recently been used to attach to several areas of a body surface to enhance massage, therapy, and exercise effects. The electrical muscle stimulation device electrically stimulates the muscles adjacent to the attached area and uses muscle contraction to increase muscular strength of the user, promote blood circulation, relieve fatigue, and provide massage without any additional exercise or strain on the joints.

In conventional electrical muscle stimulation devices, control signals are transmitted to a plurality of electrode pads through a single channel, so that it is difficult to focus electrical stimulation on a specific body part desired by the user. In addition, because electrical muscle stimulation is limited to the attachment location of the electrode pad, it is difficult to effectively provide electrical stimulation to a body area to which an electrode pad is not attached, and the location at which electrical muscle stimulation occurs cannot be varied.

### [DETAILED DESCRIPTION OF THE INVENTION]

### [TECHINICAL PROBLEM]

An aspect of the present disclosure provides an electrical muscle stimulation system and an electrical muscle stimulation method capable of controlling a variable channel, by which control signals are transmitted to a plurality of electrode channels through individual channels, so that electrical muscle stimulation is applied to a specific human body part.

In addition, another aspect of the present disclosure provides an electrical muscle stimulation system and an electrical muscle stimulation method capable of effectively providing electrical stimulation to a human body area to which an electrode pad is not attached and varying the occurring location of the electrical muscle stimulation.

Objects of the present disclosuret may not be limited to the above, and other objects not mentioned herein will be clearly understandable to those having ordinary skill in the art from the following disclosures.

### [TECHNICAL SOLUTION]

According to an embodiment, an electrical muscle stimulation system includes a main body provided to be wearable by a user or to be able to be in contact with a body of the user, a plurality of electrode pads provided on the main body to be spaced apart from each other at locations corresponding to various human body parts, and a control unit that outputs a plurality of control signals to the plurality of electrode pads through a plurality of individual channels to individually control the plurality of electrode pads according to the plurality of control signals.

The control unit may determine the control signal by selecting one or more working electrode pads from among the plurality of electrode pads according to a target location and a target intensity of electrical muscle stimulation to be caused to the body of the user.

The plurality of electrode pads may selectively operate as the working electrode pad according to the control signal transmitted through the individual channel, and the working electrode pad may generate an electrical signal for electrical muscle stimulation according to the control signal.

The control unit may determine the plurality of control signals according to a type of the user and at least one of the human body parts to which the electrical muscle stimulation is applied, and determine the plurality of control signals differently for each of a plurality of preset setting modes.

The control unit may transmit the control signal to the plurality of electrode pads through a plurality of channels, and adjust an frequency and an intensity of the electrical signal by independently generating the control signal for each channel corresponding to the plurality of setting modes.

The control unit may select a plurality of working electrode pads for generating the electrical signal from among the plurality of electrode pads according to the target location and the target intensity of the electrical muscle stimulation, and determine a frequency and an intensity of the electrical signal according to locations of the plurality of working electrode pads, the target location, and the target intensity.

The control unit may set an electrical muscle stimulation area according to the target location and the target intensity of the electrical muscle stimulation, and select electrode pads located within the electrical muscle stimulation area from among the plurality of electrode pads as the working electrode pads.

The control unit may extract a plurality of electrode pad combinations for three or more electrode pads located within the electrical muscle stimulation area, analyze an occurring location and an occurring intensity of electrical muscle stimulation for each of the plurality of electrode pad combinations, and determine an electrode pad combination having the occurring location and the occurring intensity that best matches the target location and the target intensity among the plurality of electrode pad combinations to select the electrode pad combination as the working electrode pad.

According to another embodiment, an electrical muscle stimulation method includes individually controlling, by a control unit, a plurality of electrode pads according to a plurality of control signals by outputting the plurality of control signals to the plurality of electrode pads through a plurality of individual channels, wherein the plurality of electrode pads is provided on a main body to be spaced apart from each other at locations corresponding to various human body parts, and the main body is provided to be wearable by a user or to be able to be in contact with a body of the user.

The controlling of the plurality of electrode pads may include determining the control signal by selecting one or more working electrode pads from among the plurality of electrode pads according to a target location and a target intensity of electrical muscle stimulation to be caused to the body of the user, and generating, by a working electrode pad, an electrical signal for electrical muscle stimulation according to the control signal, wherein, among the plurality of electrode pads, the working electrode pad may selectively operate according to the control signal transmitted through the individual channel.

The controlling of the plurality of electrode pads may include determining the plurality of control signals according to a type of the user and at least one of the human body parts to which the electrical muscle stimulation is applied, and determining the plurality of control signals differently for each of a plurality of preset setting modes.

The controlling of the plurality of electrode pads may include transmitting the control signal to the plurality of electrode pads through a plurality of channels, and adjusting an frequency and an intensity of the electrical signal by independently generating the control signal for each channel corresponding to the plurality of setting modes.

The controlling of the plurality of electrode pads may include selecting a plurality of working electrode pads for generating the electrical signal from among the plurality of electrode pads according to the target location and the target intensity of the electrical muscle stimulation, and determining a frequency and an intensity of the electrical signal according to locations of the plurality of working electrode pads, the target location, and the target intensity.

The controlling of the plurality of electrode pads may include setting an electrical muscle stimulation area according to the target location and the target intensity of the electrical muscle stimulation, and selecting electrode pads located within the electrical muscle stimulation area from among the plurality of electrode pads as the working electrode pads.

The controlling of the plurality of electrode pads may include extracting a plurality of electrode pad combinations for three or more electrode pads located within the electrical muscle stimulation area, analyzing an occurring location and an occurring intensity of electrical muscle stimulation for each of the plurality of electrode pad combinations, and determining an electrode pad combination having the occurring location and the occurring intensity that best matches the target location and the target intensity among the plurality of electrode pad combinations to select the electrode pad combination as the working electrode pad.

The controlling of the plurality of electrode pads may include comparing distances between the target location of the electrical muscle stimulation and the plurality of working electrode pads with electrode distances between the plurality of working electrode pads, respectively, predicting energy of an electrical pulse transmitted from each working electrode pad to the target location according to propagation characteristics of the electrical pulse in consideration of human tissue characteristics between each working electrode pad and the target location; and determining a control signal to be caused to the plurality of working electrode pads by attenuating at least one of an electric pulse amplitude and an electric pulse frequency generated from each working electrode pad from a target pulse amplitude or a target pulse frequency by reflecting a ratio of the distances to the electrode distance and the propagation characteristics of the electrical pulse.

According to still another embodiment, there is provided a computer-readable non-transitory recording medium on which a program for executing the electrical muscle stimulation method is recorded.

### [ADVANTAGEOUS EFFECTS OF THE INVENTION]

According to the embodiments of the present disclosure, there are provided an electrical muscle stimulation system and an electrical muscle stimulation method capable of controlling a variable channel, by which control signals are transmitted to a plurality of electrode channels through individual channels, so that electrical muscle stimulation is applied to a specific human body part.

In addition, according to the embodiments of the present disclosure, there are provided an electrical muscle stimulation system and an electrical muscle stimulation method that are capable of effectively transmitting electrical stimulation to body areas where electrode pads are not attached and varying the location of electrical muscle stimulation.

Effects obtained by various embodiments of the disclosure may not be limited to the above, and other effects will be clearly understandable to those having ordinary skill in the art from the following disclosures.

### [DESCRIPTION OF THE DRAWINGS]

FIG. 1 is a diagram illustrating an electrical muscle stimulation system according to an embodiment of the present disclosure.
FIG. 2 is a diagram illustrating an electrical muscle stimulation system according to another embodiment of the present disclosure.
FIG. 3 is a diagram schematically illustrating a state of use of the electrical muscle stimulation system according to the embodiment of FIG. 2.
FIG. 4 is a diagram illustrating a bathtub constituting the electrical muscle stimulation system of FIG. 2 viewed from above.
FIG. 5 is a diagram illustrating an electrical muscle stimulation system according to an embodiment of the present disclosure.
FIG. 6 is a flowchart illustrating an electrical muscle stimulation method according to an embodiment of the present disclosure.
FIG. 7 is a flowchart illustrating an electrical muscle stimulation method according to an embodiment of the present disclosure.
FIG. 8 is a flowchart illustrating operation S40 of FIG. 7.
FIG. 9 is a diagram illustrating an electrical muscle stimulation method according to an embodiment of the present disclosure.
FIG. 10 is a flowchart illustrating an electrical muscle stimulation method according to another embodiment of the present disclosure.
FIG. 11 is a diagram illustrating an electrical muscle stimulation method according to the embodiment of FIG. 10.

### [BEST MODE]

Advantages and features of embodiments of the present disclosure, and method for achieving thereof will be apparent with reference to the accompanying drawings and detailed description that follows. But, it should be understood that the present disclosure is not limited to the following embodiments and may be embodied in different ways, and that the embodiments are given to provide complete disclosure of the present disclosure and to provide thorough understanding of the present disclosure to those skilled in the art, and the scope of the present disclosure is limited only by the accompanying claims and equivalents thereof.

Even though it is not defined, all terms (including technical or scientific terms) used herein have the same meanings as those belonging to the present disclosure is generally accepted by common techniques in the art. The terms defined in general dictionaries may be construed as having the same meanings as those used in the related art and/or a text of the present application and even when some terms are not clearly defined, they should not be construed as being conceptual or excessively formal.

Like reference numerals refer to like elements throughout the specification. This specification does not describe all the elements of the embodiments, and the general contents of the related art or duplicative contents in the embodiments will be omitted. The term "~ unit", as used in the specification, may refer to a unit that processes at least one function or operation, and may be implemented, for example, as software, FPGA, or hardware.

The functions provided by "- unit" may be performed separately by multiple components or integrated with other additional components and multiple "- units" may be implemented as one component. However, the term "- unit" is not limited to software or hardware. The "- unit" may be configured to be included in an addressable storage medium and to play one or more processors.

Terms, such as "first", "second", etc, are for discriminating one component from another component, but the scope is not limited to the terms. Singular forms are intended to include plural forms unless the context clearly indicates otherwise. In the present disclosure, the term "and/or" indicates each of listed components or various combinations thereof.

The reference numeral assigned to each operation constituting a method according to an embodiment of the present disclosure are used only for convenience of explanation, and do not indicate the order of each operation, and do not clearly state a specific order in the context. Unless otherwise specified, it may be carried out differently from the specified order. Hereinafter, the working principle and embodiments of the present disclosure will be described with reference to the accompanying drawings.

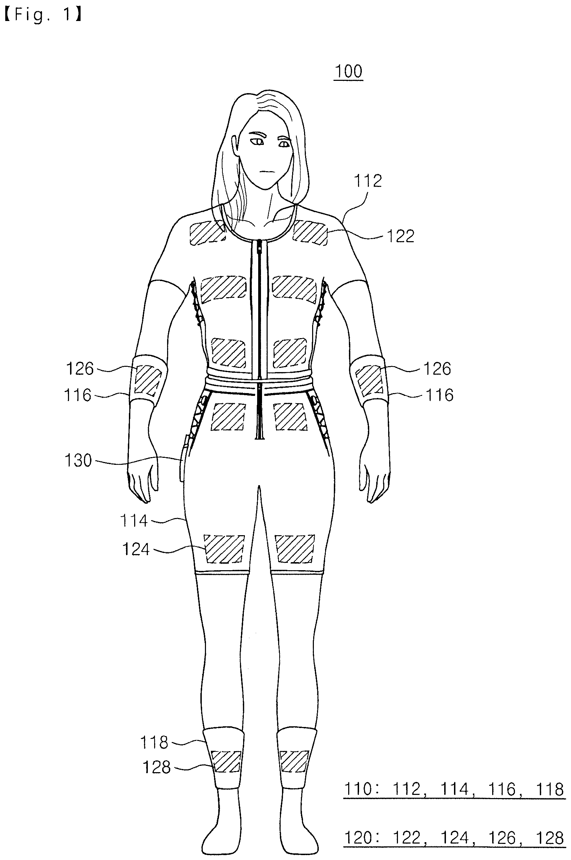

FIG. 1 is a diagram illustrating an electrical muscle stimulation system according to an embodiment of the present disclosure. Referring to FIG. 1, an electrical muscle stimulation system 100 according to an embodiment of the present disclosure may include a main body 110, a plurality of electrode pads 120 provided on the main body 110, and a control device 130 that controls the plurality of electrode pads 120 to generate an electrical signal for electrical muscle stimulation through at least one of the plurality of electrode pads 120.

The electrical muscle stimulation system 100 may be used in a state where a user wears the electrical muscle stimulation system 100 in the form of a suit, band, patch, or the like in the air or in water, or may be used in a spa form in a bathtub. In a state where a user wears a suit, band, patch, or the like that constitutes the electrical muscle stimulation system 100 in the air or in water, or in a spa-like form in a bathtub equipped with the plurality of electrode pads 120, the user may receive electrical muscle stimulation by the electrical muscle stimulation system 100 to receive health care or rehabilitation treatment, or may be provided with human sensory stimulation implemented by a virtual reality system and/or an augmented reality system such as a virtual reality (VR)/augmented reality (AR) game.

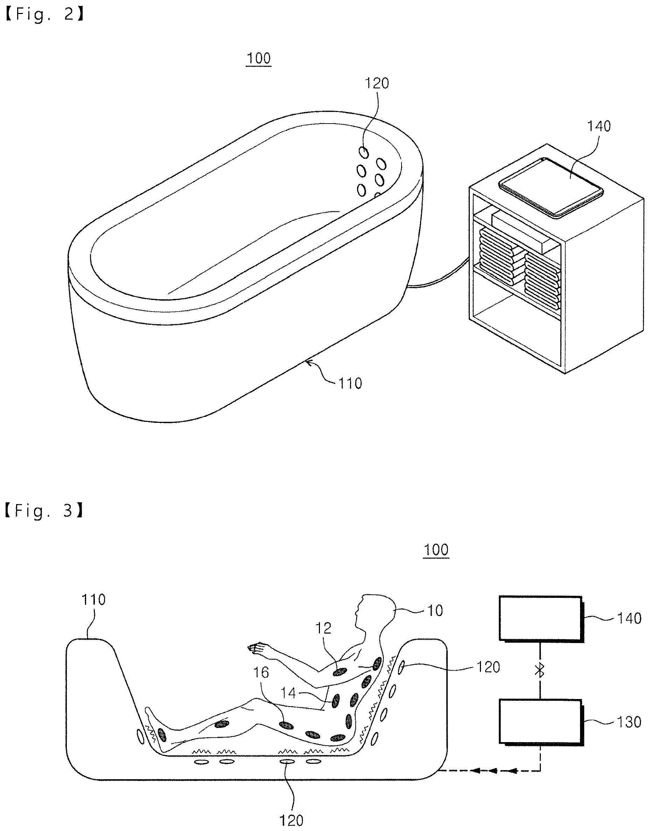

The main body 110, which is a component that supports the electrode pad 120, may be provided to be worn by the user or to be in contact with the human body of the user. The main body 110 may be provided in the form of a suit, band, patch, or the like that can be worn by the user. Alternatively, as in the embodiments of FIGS. 2 to 4 described later, the main body 110 may be provided in a bathtub-like form such that the user receives electrical muscle stimulation in a spa form.

In the embodiment of FIG. 1, the main body 110 may include an upper suit 112 that allows the user to wear it on the upper body, a lower suit 114 that allows the user to wear it on the lower body, and one or more bands 126 and 128 that are provided to be worn by wrapping it around a body part such as an arm, a leg, and an abdomen, and the like. Alternatively, the main body 110 may be provided in the form of a patch attached to the user's body.

The plurality of electrode pads 120 may be provided on one or more main bodies 110 and spaced apart from each other at locations corresponding to various body parts of the user. As an example, the electrode pad 120 may include one or more electrode pads 122 provided on the upper suit 112, one or more electrode pads 124 provided on the lower suit 114, and one or more electrode pads 126 and 128 provided on one or more bands 126 and 128 worn on an arm, a leg, or the like.

The electrode pad 120 may be formed of a conductive material, and may be arranged on an inside surface of the main body 110, which is in contact with the user's body while the user wears the main body 110, in order to effectively transmit electrical stimulation to the user's muscles. Each electrode pad 120 may selectively operate as a working electrode pad according to a control signal transmitted from the control device 130 through an individual channel. The electrode pad 120, which operates as a working electrode pad, may generate an electrical signal to apply electrical stimulation to the user's muscles according to the control signal received from the control device 130.

The control device 130 may be provided in the main body 110 or may be provided outside the main body 110. The control device 130 may be connected to each electrode pad 120 through wired or wireless communication. The control device 130 may include a control unit. The control unit may include at least one processor. The control unit may output a plurality of control signals to the plurality of electrode pads 120 through a plurality of individual channels. The control unit may individually control the plurality of electrode pads 120 according to the plurality of control signals transmitted through the plurality of individual channels.

As an example, the control signal output from the control unit may be an electrical signal. That is, an electrical signal output unit (not shown) may be provided in the control unit, and a control signal may be transmitted in the form of an electrical signal to the electrode pad 120 through an individual channel and output through the electrode pad 120. In this case, the control signal may be transmitted to the electrode pad 120 in the form of an electric pulse having a pulse amplitude and pulse frequency.

As another example, the control signal output from the control unit may be transmitted to the electrode pad 120 as an electrode control signal rather than an electric pulse. In this case, the control signal output from the control unit may include pulse amplitude information and pulse frequency information of the electrical signal to be output through the electrode pad 120, or may include information for identifying the electrical pulse of the electrical signal. The electrode pad 120 may be provided with an electrical signal output unit that generates and outputs an electric pulse according to a control signal received from the control unit.

The control device 130 may be provided with an input unit (not shown) that allows the user to input the target location and target intensity of electrical muscle stimulation. When the target location and target intensity of the electrical muscle stimulation to be caused to the user's human body are input by the input unit of the control device 130, the control unit may select at least one working electrode pad from the plurality of electrode pads 120 according to the target location and target intensity of the input electrical muscle stimulation, and determine a control signal to be applied to the selected working electrode pad.

In an embodiment, the user may select at least one from various body parts such as shoulder, chest, neck, arm, wrist, waist, thigh, calf, and the like through the input unit and input the target location and target intensity. As another example, the user may determine the target location of the electrical muscle stimulation by inputting a location at which the user desires to receive or concentrate electrical muscle stimulation in such a manner that the location is touched in a human body image displayed on the input unit.

The control unit of the control device 130 may select one or more working electrode pads from the plurality of electrode pads 120, which match the target location and target intensity of the electrical muscle stimulation input by the user through the input unit. To this end, location information at where each electrode pad 120 is placed while the user wears the main body 110 may be set in advance in the storage unit of the control device 130.

The electrode pad 120 may generate an electrical signal according to a control signal transmitted from the control unit of the control device 130 to provide electrical stimulation to the user's muscles corresponding to the target location of the electrical muscle stimulation. The current intensity and/or frequency of the electrical signal generated from the electrode pad 120 may be adjusted according to the control signal transmitted from the control unit through an individual channel.

The control device 130 may individually (independently) set the level (current intensity level and/or frequency level) of the electrical signal generated for each part through the electrode pad 120. The control device 130 may further include a notification unit (not shown). The notification unit may perform the function of informing the user of various electrical muscle stimulation information such as the location and intensity of the electrical stimulation applied to the user. The scheme by which the notification unit notifies the user of information may be any scheme, such as a scheme of displaying information on a display screen or a scheme of outputting sound through a speaker.

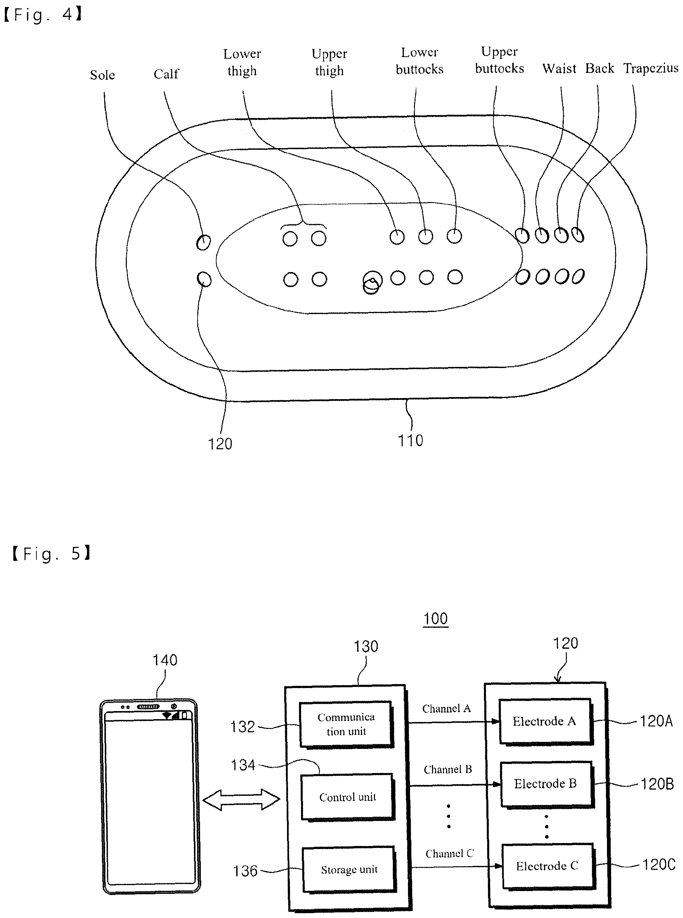

FIG. 2 is a diagram illustrating an electrical muscle stimulation system according to another embodiment of the present disclosure. FIG. 3 is a diagram schematically illustrating a state of use of the electrical muscle stimulation system according to the embodiment of FIG. 2. FIG. 4 is a diagram illustrating a bathtub constituting the electrical muscle stimulation system of FIG. 2 viewed from above.

Referring to FIGS. 2 to 4, the electrical muscle stimulation system 100 according to an embodiment of the present disclosure is different from the embodiment in FIG. 1 in that it is configured to allow the user to receive electrical muscle stimulation in the form of a spa while the user is underwater in a bathtub. The electrical muscle stimulation system 100 includes the main body 110 implemented in the form of a bathtub, the plurality of electrode pads 120 distributed and disposed on the main body 110, the control device 130, and a user terminal 140.

Bathtubs may be installed in various facilities such as homes, lodging facilities, and businesses and be replaced with various items that collect water and a person can enter. The electrode pad 120 may be provided in an area in contact with the human body among inner areas of the bathtub-shaped main body 110. The electrode pad 120 may stimulate the user's motor nerves by applying electrical stimulation to the human body by outputting an electrical pulse according to a control signal in the form of an electrical signal provided by the control device 130. Thus, the user may strengthen the body muscles or achieve a massage effect.

For example, the electrode pad 120 may be proved in an area that contacts or is adjacent to at least one of the trapezius, back, waist, upper buttocks, lower buttocks, upper thighs, lower thighs, calves, or soles of a person when the person lies in a bathtub. The bathtub may have a built-in battery for operating the electrode pad 120, and may be connected to the control device 130 or an external power source through a wire.

The control unit of the control device 130 may include an oscillation unit. The oscillation unit of the control device 130 may generate an electrical pulse through the electrode pad 120 based on control data, and generate an electrical pulse of various amplitudes and frequencies depending on the purpose of massage, muscle strengthening, or electrical stimulation location.

For example, the oscillation unit may include a pulse generation unit capable of generating an electrical pulse, a display unit capable of displaying a pulse output mode or pulse output intensity, and a control unit capable of controlling the pulse output mode or pulse output intensity. The control device 130 may generate control data for controlling an electrical pulse, for example, data for controlling the level of a pulse, such as a current intensity, a voltage level (pulse amplitude) or a frequency (pulse frequency) constituting the pulse.

The oscillation unit may generate an electrical pulse whose amplitude and frequency are controlled based on control data generated by the control device 130, and the generated electrical pulse may be output through the electrode pad 120. The control device 130 may receive a control command for controlling the level of an electrical pulse, such as the amplitude or frequency of the electrical pulse, from the user and then generate control data based on the control command. The control data generated in such a manner may be used to control the electrical pulse generated from the oscillation unit.

The user terminal 140 may be provided as a device such as a smartphone, a tablet, a PC, or the like. An application capable of controlling the electrical muscle stimulation system 100 may be stored in a storage medium within the user terminal 140. A user of the electrical muscle stimulation system 100 may execute an application installed on the user terminal 140 and then input a control command. The user terminal 140 may generate control data based on the received control command and transmit the control data to the control device 130 through wired/wireless communication. The electrical pulse generated through the oscillation unit of the control device 130 may be controlled based on the control data transmitted from the user terminal 140.

The control command may be a command to output electrical pulses with different amplitudes and frequencies for each electrode pad 120 corresponding to the target locations 12, 14 and 16 of various electrical muscle stimulation. For example, the control command is a command to output a first level electrical pulse from the electrode pad 120 corresponding to the thigh location, and to output a second level electrical pulse from the electrode pad 120 corresponding to the waist location.

The control data may be data that causes an electrical pulse of the same level as the control command to be generated through the oscillation unit of the control device 130. In more detail, the first control data, like the control command, may be data that causes the first level electrical pulse to be output from the electrode pad 120 corresponding to the thigh location, and the second level electrical pulse to be output from the electrode pad 120 corresponding to the waist location.

FIG. 5 is a diagram illustrating an electrical muscle stimulation system according to an embodiment of the present disclosure. Referring to FIG. 5, the plurality of electrode pads 120 (120A, 120B and 120C) may selectively operate as working electrode pads according to control signals transmitted through individual channels (channel A, channel B, and channel C). In accordance with control signals provided from the control device 130 through individual channels (channel A, channel B, and channel C), the working electrode pads among the plurality of electrode pads 120 may generate electrical signals for electrical muscle stimulation.

The control device 130 may include a communication unit 132 that communicates with the user terminal 140 and the electrode pad 120, a control unit 134 that generates a control signal to be provided to the electrode pad 120 and outputs the control signal to the electrode pad 120 through an individual channel, and a storage unit 136 that stores data related to electrical muscle stimulation.

The control unit 134 may generate a wired control signal and/or a wireless control signal that controls the operation of the electrical muscle stimulation system 100. The communication unit provided in the control device 130 may communicate with the user terminal 140 through a wired/wireless communication network and may transmit the wired/wireless control signal to the electrode pad 120 provided in a suit, a bathtub, or the like.

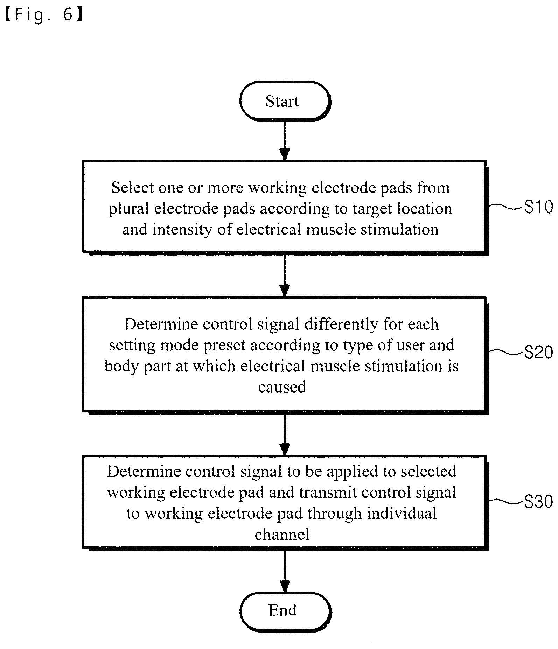

FIG. 6 is a flowchart illustrating an electrical muscle stimulation method according to an embodiment of the present disclosure. Referring to FIGS. 5 and 6, in operation S10, the control unit 134 may select a plurality of working electrode pads for generating an electrical signal from among the plurality of electrode pads 120 according to the target location and target intensity of the electrical muscle stimulation.

In operation S20, the control unit 134 may determine a plurality of control signals differently for each setting mode preset according to the type of user (gender, age group, body type, and the like), the body part at which electrical muscle stimulation is caused, and the type of electrical muscle stimulation (exercise, rehabilitation, fatigue recovery, massage, and the like). For example, the plurality of setting modes may be set for various body parts, such as chest, shoulders, back, waist, thighs, arms, and the like.

In operation S30, the control unit 134 may determine a control signal to be applied to a working electrode pad selected from among the plurality of electrode pads 120 and transmit the control signal to the working electrode pad through an individual channel. Accordingly, it is possible to independently generate control signals for each channel corresponding to the plurality of setting modes and adjust the frequency and intensity of the electrical signal output through the plurality of working electrode pads.

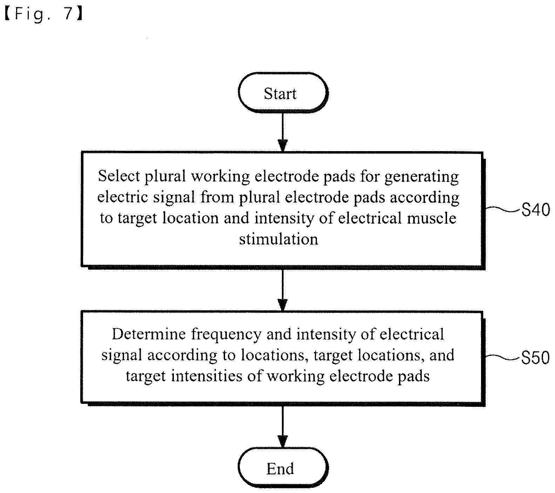

FIG. 7 is a flowchart illustrating an electrical muscle stimulation method according to an embodiment of the present disclosure. Referring to FIGS. 5 and 7, in operation S40, the control unit 134 may select one or more working electrode pads from the plurality of electrode pads according to the target location and target intensity of the electrical muscle stimulation to be caused in the user's body. In operation S50, the control unit 134 may determine the frequency and intensity of the electrical signal according to the locations, target locations, and target intensities of the plurality of working electrode pads.

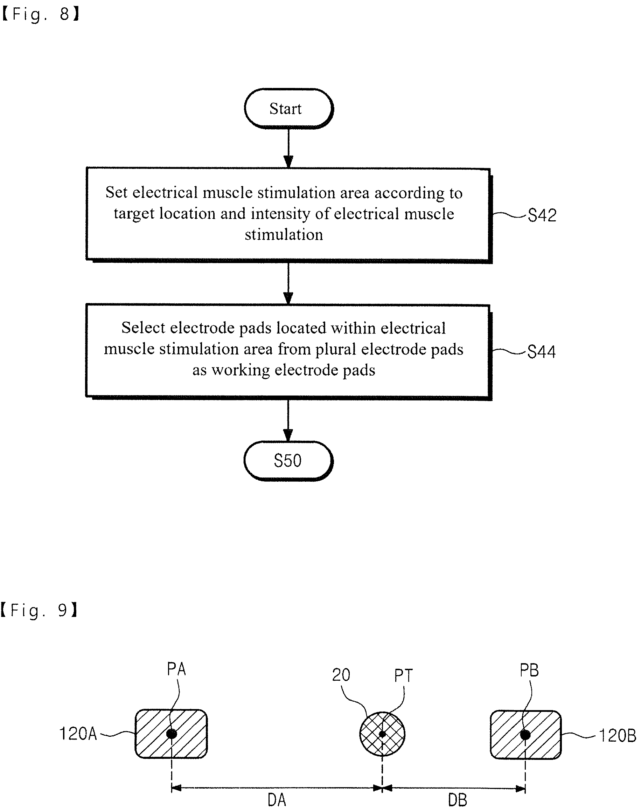

FIG. 8 is a flowchart illustrating operation S40 of FIG. 7. FIG. 9 is a diagram illustrating an electrical muscle stimulation method according to an embodiment of the present disclosure. Referring to FIGS. 5 and 7 to 9, in operation S42, the control unit 134 may set an electrical muscle stimulation area within a set distance centered on a target location PT according to the target location PT and target intensity of the electrical muscle stimulation. The electrical muscle stimulation area may be set to, for example, a circle, a sphere, a square, or the like. In operation S44, the control unit 134 may select electrode pads located within the electrical muscle stimulation area from the plurality of electrode pads as working electrode pads.

In the example of FIG. 9, the control unit 134 may select two electrode pads located within the electrical muscle stimulation area set according to the target location and target intensity of an electrical muscle stimulation 20 as working electrode pads 120A and 120B. The control unit 134 may determine a control signal to be applied to the working electrode pads 120A and 120B in order to apply the electrical muscle stimulation 20 to the target location PT.

The control unit 134 may determine the stimulation intensity and frequency of the control signal to be applied to the working electrode pads 120A and 120B according to distances DA and DB between the target location PT of the electrical muscle stimulation 20 and the working electrode pads 120A and 120B. For example, the control unit 134 may attenuate the stimulation intensity and/or frequency in proportion to the distances DA and DB between the target location PT of the electrical muscle stimulation 20 and the working electrode pads 120A and 120B in order to determine the control signal to be applied to the working electrode pads 120A and 120B.

For example, the control unit 134 may compare the first distance DA between the target location PT of the electrical muscle stimulation 20 and the first working electrode pad 120A with the electrode distance between the first working electrode pad 120A and the second working electrode pad 120B, and may attenuate the electric pulse amplitude generated from the first working electrode pad 120A from the target pulse amplitude (target intensity) to a first pulse amplitude by reflecting the ratio of the first distance DA to the electrode distance.

In addition, the control unit 134 may compare the second distance DB between the target location PT of the electrical muscle stimulation 20 and the second working electrode pad 120B with the electrode distance between the first working electrode pad 120A and the second working electrode pad 120B, and may attenuate the electric pulse amplitude generated from the second working electrode pad 120B from the target pulse amplitude (target intensity) to a second pulse amplitude by reflecting the ratio of the second distance DB to the electrode distance.

When an electrical pulse with a first pulse amplitude is output from the first working electrode pad 120A and an electrical pulse with a second pulse amplitude is output from the second working electrode pad 120B, an electrical pulse corresponding to the target intensity (target amplitude) may be induced at the target location PT of the electrical muscle stimulation 20, and the effects of exercise, rehabilitation, massage, and the like may be concentrated on the specific body part desired by the user.

As another example, the control unit 134 may compare the first distance DA between the target location PT of the electrical muscle stimulation 20 and the first working electrode pad 120A with the electrode distance between the first working electrode pad 120A and the second working electrode pad 120B, and may attenuate the frequency of the electric pulse generated from the first working electrode pad 120A from the target pulse frequency (target pulse energy) to a first pulse frequency by reflecting the ratio of the first distance DA to the electrode distance.

In addition, the control unit 134 may compare the second distance DB between the target location PT of the electrical muscle stimulation 20 and the second working electrode pad 120B with the electrode distance between the first working electrode pad 120A and the second working electrode pad 120B, and may attenuate the frequency of the electric pulse generated from the second working electrode pad 120B from the target pulse frequency (target pulse energy) to a second pulse frequency by reflecting the ratio of the second distance DB to the electrode distance.

When an electrical pulse with a first pulse amplitude is output from the first working electrode pad 120A and an electrical pulse with a second pulse amplitude is output from the second working electrode pad 120B, an electrical pulse corresponding to the target intensity (target energy) may be induced at the target location PT of the electrical muscle stimulation 20, and the effects of exercise, rehabilitation, massage, and the like may be concentrated on the specific body part desired by the user.

In addition, the control unit 134 may complexly adjust the pulse amplitude and pulse frequency according to the locations of the working electrode pads 120A and 120B and the target location PT of the electrical muscle stimulation in order to determine the control signal that causes electrical muscle stimulation corresponding to the target intensity at the target location PT by the generated electrical pulse.

In this case, the control unit 134 may attenuate the electrical pulse energy generated from each of the working electrode pads 120A and 120B in proportion to the working electrode pads 120A and 120B and the target location PT, and may determine the amplitude and frequency of the electric pulse generated from the working electrode pads 120A and 120B such that the sum of the electrical pulse energies generated from the working electrode pads 120A and 120B and transmitted to the target location PT is equal to the target pulse energy.

According to an embodiment of the present disclosure, the control unit 134 may predict the energy of the electric pulse transmitted to the target location PT according to the propagation characteristics of the electrical pulse, considering not only the distance between the working electrode pads 120A and 120B and the target location PT for electrical muscle stimulation but also characteristics of human tissue (fat, muscle ratio, bone, and the like) between the working electrode pads 120A and 120B and the target location PT for electrical muscle stimulation.

In this case, the human tissue or propagation characteristics of electrical pulses may be set in advance for each body area. For example, the human tissue/propagation characteristics of electrical pulses by each body area may be determined by adding the electric pulse propagation characteristics defined by tissue type such as fat, muscle, and bone by reflecting the ratio of fat, muscle, and bone in each body area.

In the embodiment of FIG. 9, the two working electrode pads 120A and 120B are described as an example, but the control signal may be applied to three or more working electrode pads through individual channels to induce the target electrical pulse at the target location PT. In this case, the propagation of the electrical pulse may be analyzed in each three-dimensional direction to determine control signals applied to three or more working electrode pads to satisfy all target electrical muscle stimulation conditions for each direction.

The embodiment of FIG. 9 may be applied to an application that applies realistic stimulation to the user's human body according to the impact location (location at which impact is applied) in, for example, VR/AR games or survival games. In this case, a detection unit (e.g., a pressure sensor, or the like) may be provided on a suit, band, patch, or the like, or the location of the impact received by the user may be detected through the opponent's attack information, and the like, and the detected location of the impact may be set as the target location of electrical muscle stimulation.

The working electrode pads selected from among the electrode pads 120 may be adjusted according to the magnitude of the impact and the electrical muscle stimulation area that changes accordingly. For example, when the magnitude of the impact is large, the area of stimulation that the user receives may be increased such that the number of working electrode pads is increased in proportion to the magnitude of the impact.

Meanwhile, the control unit 134 may determine the control signal (the pulse amplitude, the pulse frequency, and the like of an electrical signal) to be generated from the electrode pads 120 in order to apply electrical muscle stimulation to a specific body location of the user based on an artificial intelligence model trained based on learning data.

The target intensity (target strength) of electrical muscle stimulation may be adjusted according to the shock received by the user in a VR/AR game, a survival game, or the like. For example, in the case of a survival game, the pressure (impact) value sensed by the suit worn by the user and the degree of pain felt by the user depending on the location of the impact may be adjusted. The degree of pain experienced by the user depending on the impact location may be preset for each body part.

Embodiments of the present disclosure may be used not only for VR/AR games and survival games, but also for receiving electrical muscle stimulation for the purposes of exercise, rehabilitation, fatigue recovery, and massage on body parts where electrode pads are not located. In other words, when the user inputs an arbitrary body part for which electrical muscle stimulation is desired through the user terminal, the control unit may determine the working electrode pad and control signal accordingly, such that appropriate electrical muscle stimulation is applied to the arbitrary input body part and maximizes the effect of electrical muscle stimulation on the corresponding body part.

As described above, according to the electrical muscle stimulation method according to an embodiment of the present disclosure, the location of electrical stimulation may not be limited to the locations of the electrode pads, and electrical muscle stimulation may be applied to the user by applying electrical pulses of appropriate amplitude and/or frequency to various body locations between electrode pads.

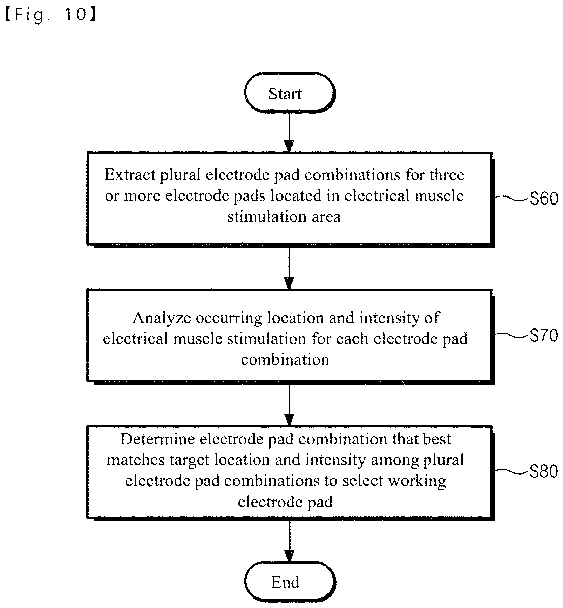

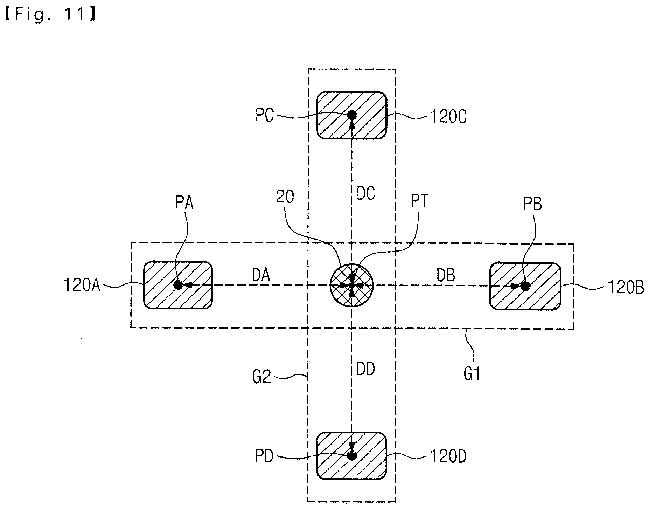

FIG. 10 is a flowchart illustrating an electrical muscle stimulation method according to another embodiment of the present disclosure. FIG. 11 is a diagram illustrating an electrical muscle stimulation method according to the embodiment of FIG. 10. Referring to FIGS. 5, 10 and 11, in operation S60, the control unit 134 may extract a plurality of electrode pad combinations for three or more electrode pads located in the electrical muscle stimulation area.

In this case, the control unit 134 may determine, as the same electrode pad combination, electrodes located in areas that are symmetrical to each other on both sides based on the target location PT of the electrical muscle stimulation 20 among three or more electrode pads located in the electrical muscle stimulation area.

Alternatively, the control unit 134 may determine an electrode pad combination of three or more electrode pads, which are part of all electrode pads located in the electrical muscle stimulation area. In this case, at least two electrode pads among the electrode pads included in the same electrode pad combination are located in areas that are symmetrical to each other on both sides based on the target location PT.

For example, in FIG. 11, the control unit 134 may extract two electrode pad combinations G1 and G2 from four electrode pads 120A, 120B, 120C, and 120D located within an electrical muscle stimulation area set according to the target location PT and target intensity of the electrical muscle stimulation 20.

In operation S70, the control unit 134 may analyze the occurring location and intensity of electrical muscle stimulation for each of a plurality of electrode pad combinations. In operation S80, the control unit 134 may determine an electrode pad combination showing the occurring location and intensity that best matches the target location and intensity among the plurality of electrode pad combinations G1 and G2 and may finally select an electric pulse as the working electrode pad to which the electrical pulse will be applied.

For example, after analyzing the electrical pulse induced at the target location PT for each electrode pad combination G1 or G2 according to the embodiment of FIG. 9, the control unit 134 may determine the electrical pulse similarity by comparing the electrical pulse analyzed to be induced at the target location PT for each electrode pad combination G1 or G2 with the target electrical pulse.

As described above, the control unit 134 may determine the stimulation intensity and frequency of the control signal to be applied to the working electrode pads 120A, 120B, 120C, and 120D according to the distances DA, DB, DC, and DD between the target location PT of the electrical muscle stimulation 20 and the working electrode pads 120A, 120B, 120C, and 120D, and thus, may compare the occurring location and intensity of electrical stimulation caused at the target location PT with the target stimulation location and intensity.

The control unit 134 may analyze the location and intensity of electrical muscle stimulation for each of the plurality of electrode pad combinations G1 and G2, and may determine one electrode pad combination (the electrode pad combination that represents the result with the highest similarity to the target pulse) that represents the occurring location and intensity that best matches the target location and intensity among the plurality of electrode pad combinations, thereby selecting the electrode pads of the corresponding electrode pad combination as the working electrode pads.

The control unit 134 may select two or more electrode pads located within the electrical muscle stimulation area set according to the target location and intensity of the electrical muscle stimulation 20 as the working electrode pads. The control unit 134 may determine the control signal to be applied to the working electrode pads in order to apply the electrical muscle stimulation 20 to the target location PT.

The electrical muscle stimulation system and electrical muscle stimulation method according to the described embodiment of the present disclosure may be implemented in the form of a program that is driven by a processor. In this case, the program may include program commands, data files, and data structures, and the like, singly or in combination. The program may be designed and produced using machine code or high-level language code.

The program may be specially designed to implement the above-described method, or may be implemented using various functions or definitions that are known and available to those skilled in the art in the field of computer software. The program for implementing the above-described method may be recorded on a recording medium readable by a processor. In this case, the recording medium may include a memory.

The memory may store programs that perform the operations described above and the operations described later, and the memory may execute the stored programs. In the case where there are multiple processors and memories, it is possible for them to be integrated into one chip or to be provided in physically separate locations. The memory may include a volatile memory such as a static random access memory (SRAM), a dynamic random access memory (DRAM), or the like for temporarily storing data.

In addition, the memory may include a non-volatile memory such as a read only memory (ROM), an erasable programmable read only memory (EPROM), an electrically erasable programmable read only memory (EEPROM), or the like for longterm storage of control programs and control data. The processor may include various logic circuits and operation circuits, process data according to a program provided from memory, and generate control signals according to the processing results.

At least one component may be added or deleted corresponding to the performance of the components described above. In addition, it will be easily understood by those skilled in the art that the mutual locations of the components may be changed corresponding to the performance or structure of the system.

As described above, the embodiments have been described with reference to the accompanying drawings. It will be apparent to those skilled in the art that various changes and modifications may be made without departing from the spirit and scope of the present disclosure. Therefore, the disclosed embodiments are illustrative and should not be construed as limiting.

## Claims

1. An electrical muscle stimulation system comprising:
a main body provided to be wearable by a user or to be able to be in contact with a body of the user,
a plurality of electrode pads provided on the main body to be spaced apart from each other at locations corresponding to various human body parts; and
a control unit configured to output a plurality of control signals to the plurality of electrode pads through a plurality of individual channels to individually control the plurality of electrode pads according to the plurality of control signals,
wherein the control unit is configured to determine the control signal by selecting one or more working electrode pads from among the plurality of electrode pads according to a target location and a target intensity of electrical muscle stimulation to be caused to the body of the user,
the plurality of electrode pads are configured to selectively operate as the working electrode pad according to the control signal transmitted through the individual channel, and
the working electrode pad is configured to generate an electrical signal for electrical muscle stimulation according to the control signal.

2. The electrical muscle stimulation system of claim 1, wherein the control unit is configured to determine the plurality of control signals according to a type of the user and at least one of the human body parts to which the electrical muscle stimulation is applied, and determine the plurality of control signals differently for each of a plurality of preset setting modes.

3. The electrical muscle stimulation system of claim 2, wherein the control unit is configured to transmit the control signal to the plurality of electrode pads through a plurality of channels, and adjust an frequency and an intensity of the electrical signal by independently generating the control signal for each channel corresponding to the plurality of setting modes.

4. The electrical muscle stimulation system of claim 1, wherein the control unit is configured to select a plurality of working electrode pads for generating the electrical signal from among the plurality of electrode pads according to the target location and the target intensity of the electrical muscle stimulation, and determine a frequency and an intensity of the electrical signal according to locations of the plurality of working electrode pads, the target location, and the target intensity.

5. The electrical muscle stimulation system of claim 4, wherein the control unit is configured to set an electrical muscle stimulation area according to the target location and the target intensity of the electrical muscle stimulation, and select electrode pads located within the electrical muscle stimulation area from among the plurality of electrode pads as the working electrode pads.

6. The electrical muscle stimulation system of claim 5, wherein the control unit is configured to extract a plurality of electrode pad combinations for three or more electrode pads located within the electrical muscle stimulation area, analyze an occurring location and an occurring intensity of electrical muscle stimulation for each of the plurality of electrode pad combinations, and determine an electrode pad combination having the occurring location and the occurring intensity that best matches the target location and the target intensity among the plurality of electrode pad combinations to select the electrode pad combination as the working electrode pad.

7. An electrical muscle stimulation method comprising:
individually controlling, by a control unit, a plurality of electrode pads according to a plurality of control signals by outputting the plurality of control signals to the plurality of electrode pads through a plurality of individual channels, wherein the plurality of electrode pads is provided on a main body to be spaced apart from each other at locations corresponding to various human body parts, and the main body is provided to be wearable by a user or to be able to be in contact with a body of the user,
wherein the controlling of the plurality of electrode pads includes:
determining the control signal by selecting one or more working electrode pads from among the plurality of electrode pads according to a target location and a target intensity of electrical muscle stimulation to be caused to the body of the user; and
generating, by a working electrode pad, an electrical signal for electrical muscle stimulation according to the control signal, wherein, among the plurality of electrode pads, the working electrode pad selectively operates according to the control signal transmitted through the individual channel.

8. The electrical muscle stimulation method of claim 7, wherein the controlling of the plurality of electrode pads includes:
determining the plurality of control signals according to a type of the user and at least one of the human body parts to which the electrical muscle stimulation is applied, and determining the plurality of control signals differently for each of a plurality of preset setting modes.

9. The electrical muscle stimulation method of claim 8, wherein the controlling of the plurality of electrode pads includes:
transmitting the control signal to the plurality of electrode pads through a plurality of channels, and adjusting an frequency and an intensity of the electrical signal by independently generating the control signal for each channel corresponding to the plurality of setting modes.

10. The electrical muscle stimulation method of claim 8, wherein the controlling of the plurality of electrode pads includes:
selecting a plurality of working electrode pads for generating the electrical signal from among the plurality of electrode pads according to the target location and the target intensity of the electrical muscle stimulation; and
determining a frequency and an intensity of the electrical signal according to locations of the plurality of working electrode pads, the target location, and the target intensity.

11. The electrical muscle stimulation method of claim 10, wherein the controlling of the plurality of electrode pads includes:
setting an electrical muscle stimulation area according to the target location and the target intensity of the electrical muscle stimulation; and
selecting electrode pads located within the electrical muscle stimulation area from among the plurality of electrode pads as the working electrode pads.

12. The electrical muscle stimulation method of claim 11, wherein the controlling of the plurality of electrode pads includes:
extracting a plurality of electrode pad combinations for three or more electrode pads located within the electrical muscle stimulation area;
analyzing an occurring location and an occurring intensity of electrical muscle stimulation for each of the plurality of electrode pad combinations; and
determining an electrode pad combination having the occurring location and the occurring intensity that best matches the target location and the target intensity among the plurality of electrode pad combinations to select the electrode pad combination as the working electrode pad.

13. The electrical muscle stimulation method of claim 11, wherein the controlling of the plurality of electrode pads includes:
comparing distances between the target location of the electrical muscle stimulation and the plurality of working electrode pads with electrode distances between the plurality of working electrode pads, respectively;
predicting energy of an electrical pulse transmitted from each working electrode pad to the target location according to propagation characteristics of the electrical pulse in consideration of human tissue characteristics between each working electrode pad and the target location; and
determining a control signal to be applied to the plurality of working electrode pads by attenuating at least one of an electric pulse amplitude and an electric pulse frequency generated from each working electrode pad from a target pulse amplitude or a target pulse frequency by reflecting a ratio of the distances to the electrode distance and the propagation characteristics of the electrical pulse.

14. A computer-readable non-transitory recording medium on which a program for executing the electrical muscle stimulation method of claim 7 is recorded.
